# EUROPEAN PATENT SPECIFICATION

(11) **EP 1 338 259 B1**
(45) Date of publication and mention of the grant of the patent: **13.09.2006**
(21) Application number: 03001975.6
(22) Date of filing: 30.01.2003
(51) Int. Cl.: A61F 9/008, A61B 18/20

(54) **Laser treatment apparatus**
Laser-Augenbehandlungs-Einrichtung
Dispositif à laser de traitement des yeux

(30) Priority: 01.02.2002 JP 2002025967
(43) Date of publication of application: 27.08.2003
(73) Proprietor: NIDEK CO., LTD, Gamagori-shi, Aichi 443-0035 (JP)
(72) Inventor: Takada, Yasutoshi, Gamagori-shi, Aichi, 443-0005 (JP); Nakamura, Hirokazu, Nishio-shi, Aichi, 444-0304 (JP); Tajitsu, Kosyu, Nukata-gun, Aichi, 444-0113 (JP); Naito, Yasuyuki, Nukata-gun, Aichi, 444-0116 (JP)
(74) Representative: Hofer, Dorothea

(56) References cited:
- EP-A- 0 729 734
- EP-A- 0 960 610
- US-A- 5 300 062

## Description

### BACKGROUND OF THE INVENTION

### 1. Field of the Invention

The present invention relates to a laser treatment apparatus for performing treatment by irradiating an affected part with a laser beam.

### 2. Description of Related Art

There is a laser treatment apparatus which is used for treatment with a treatment laser beam (hereinafter, "a treatment beam") to irradiate an affected part of the fundus of a patient's eye and others. In this type of apparatus, an aiming beam of a different wavelength (color) from the treatment beam is generally used. Such an apparatus is known from EP-A-0960610. However, the use of the aiming beam of substantially the same wavelength (color) as the treatment beam is more convenient because the transmittance property of the treatment beam can be observed and confirmed by the use of the aiming beam. For instance, if an intermediate optic media such as a crystalline lens and a vitreous body is clouded, the transmittance property of the treatment beam largely differs depending on wavelengths (colors) of the treatment beam.

### SUMMARY OF THE INVENTION

The present invention has been made in view of the above circumstances and has an object to overcome the above problems and to provide a laser treatment apparatus capable of easily and efficiently producing an aiming beam of the same wavelength (color) as that of a treatment laser beam.

Additional objects and advantages of the invention will be set forth in part in the description which follows and in part will be obvious from the description, or may be learned by practice of the invention. The objects and advantages of the invention may be realized and attained by means of the instrumentalities and combinations particularly pointed out in the appended claims.

To achieve the purpose of the invention, there is provided a laser treatment apparatus for performing treatment by irradiating an affected part with a laser beam, the apparatus including: a laser source which emits a laser beam having a wavelength in a visible wavelength region; a polarization splitting member which splits the laser beam emitted from the laser source into a P-polarized component and an S-polarized component; and a polarization combining member which combines optical axes of the split components in a predetermined positional relation.

### BRIEF DESCRIPTION OF THE DRAWINGS

The accompanying drawings, which are incorporated in and constitute a part of this specification illustrate an embodiment of the invention and, together with the description, serve to explain the objects, advantages and principles of the invention.

In the drawings,
Fig. 1 is a perspective external view of a laser treatment apparatus in an embodiment according to the present invention;
Fig. 2 is a schematic view showing an optical system provided in the interior of the apparatus;
Fig. 3 is a block diagram showing a control system of the apparatus;
Fig. 4 is a schematic view showing a modification example of the optical system of the apparatus; and
Fig. 5 is a schematic view showing another modification example of the optical system of the apparatus.

### DETAILED DESCRIPTION OF THE PREFERRED EMBODIMENTS

A detailed description of a preferred embodiment of a laser treatment apparatus embodying the present invention will now be given referring to the accompanying drawings.

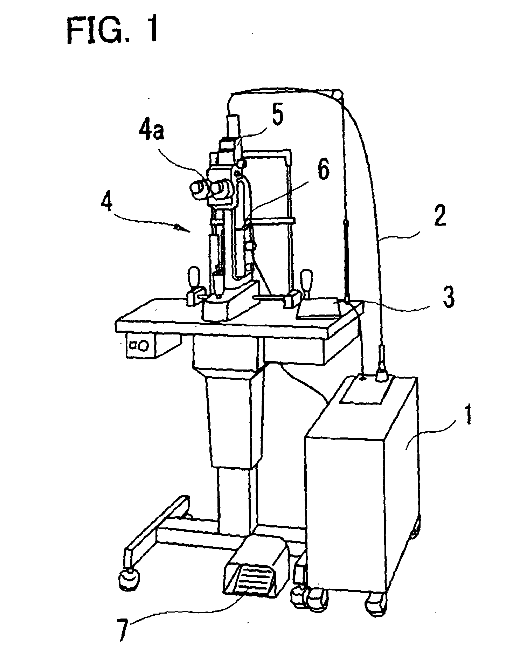

Fig. 1 is a perspective external view of a laser photocoagulation apparatus in the present embodiment. Numeral 1 is a main unit of the apparatus in which a laser source and an optical system for allowing a laser beam to be incident on an optical fiber 2. Numeral 3 is a control box for setting and displaying photocoagulation conditions (laser irradiation conditions) such as laser output power, an irradiation duration and a wavelength of the laser beam, and displaying the status of the apparatus. Numeral 4 is a slit lamp delivery for irradiating the laser beam to an affected part of a patient's eye while allowing an operator to observe the patient's eye. This slit lamp delivery 4 is provided with a laser irradiating part 5 for irradiating the laser beam delivered through the optical fiber 2, an illuminating part 6 for illuminating the patient's eye, and a binocular microscope 4 for observation of the patient's eye. Numeral 7 is a footswitch for generating a trigger signal for laser irradiation.

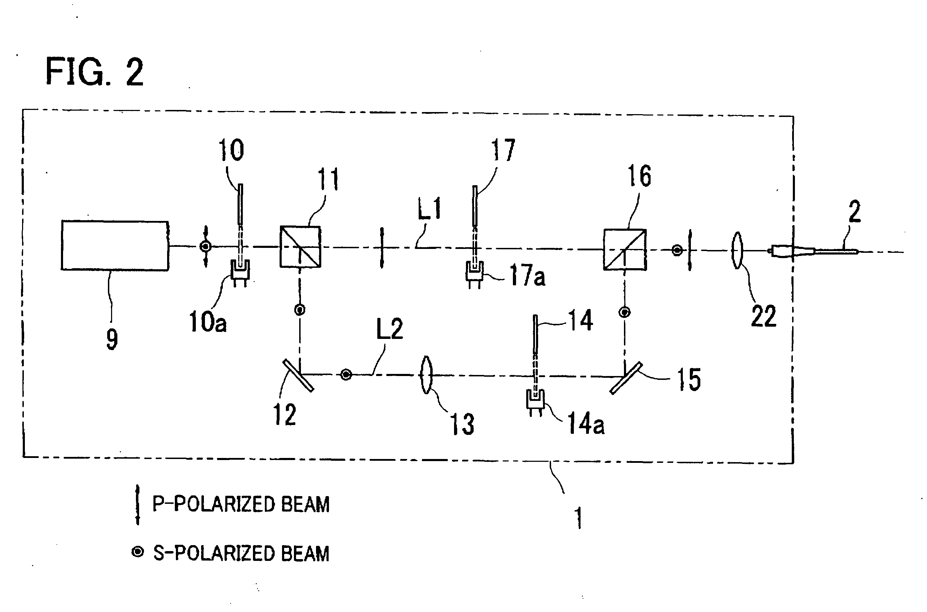

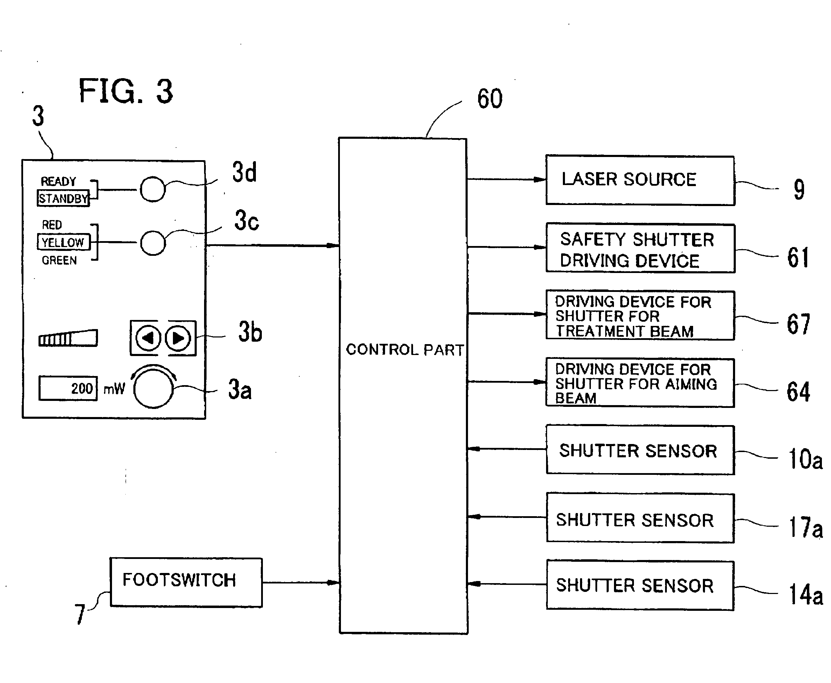

Fig. 2 is a schematic view explaining an optical system provided in the interior of the main unit 1 of the apparatus. Fig. 3 is a block diagram of a control system of the apparatus. Numeral 9 is a laser source, which is internally provided with an Nd:YAG crystal serving as a solid laser medium, a diode laser serving as an exciting light source, and a nonlinear crystal serving as a wavelength converter. The Nd:YAG crystal emits light beams having a plurality of different oscillation lines (peak wavelengths) in a near-infrared region by excitation light from the diode laser. The nonlinear crystal is used to generate the second harmonic waves of three oscillation lines of about 1064 nm, about 1123 nm, and about 1319 nm, which are higher in output power among the plurality of oscillation lines, thus emitting laser beams of three colors having wavelengths in a visible region, namely, about 532 nm (green), about 561 nm (yellow), and about 659 nm (red).

Numeral 10 is a safety shutter, which is removed from an optical path by driving of a driving device 61 to allow the laser beam to travel along the optical path and, alternatively, which is inserted in the optical path in a predetermined case for example of occurrence of an abnormal event to intercept the laser beam. The opening and closing of this safety shutter 10 is detected by a shutter sensor 10a.

Numeral 11 is a polarizer, e.g., a polarization beam splitter, which splits the laser beam from the laser source 9 into a P-polarized component and an S-polarized component. In many cases, the laser sources to be used for treatment emit linearly polarized light having a P-to-S polarization ratio of about 1000 to 1. Thus, an S-polarized component of about 1/1000 can be taken out, so that a quantity of light needed for the aiming beam can be divided with a low loss.

The P-polarized component utilized as a treatment laser beam (hereinafter, "a treatment beam") passes through the polarizer 11 and succeedingly travels along an optical axis L1. On this optical axis L1, a shutter 17 for the treatment beam is disposed. This shutter 17 is inserted in the optical path by driving of a driving device 67 to intercept the treatment beam when the treatment beam is not required. The opening and closing of the shutter 17 is detected by a shutter sensor 17a.

The S-polarized component utilized as the aiming beam is reflected by the polarizer 11 and a mirror 12 in sequence and then travels along an optical axis L2. On this optical axis L2, there is disposed a compensating lens 13 for compensating a difference in optical length between the optical axes L1 and L2. Preferably, a shutter 14 for the aiming beam is also provided on the optical axis L2. When the aiming beam is not required, the shutter 14 is inserted in the optical path by driving of a driving device 64 to intercept the aiming beam. The opening and closing of the shutter 14 is detected by a shutter sensor 14a. The S-polarized component having passed through the shutter 14 is reflected by a mirror 15 toward a polarizer 16 which combines the P-polarized beam and the S-poiaxized beam again into a coaxial beam.

The polarizer 16 allows the P-polarized beam traveling along the optical axis L1 to pass through, while reflects the S-polarized beam traveling along the optical axis L2, thereby producing a combined laser beam. Numeral 22 is a light condensing lens, which converges the laser beam on an incident end of the optical fiber 2 and allows the laser beam to be incident thereon. The laser beam delivered into the slit lamp delivery 4 through the optical fiber 2 is irradiated by the laser irradiating part 5 to an affected part of a patient's eye.

In Fig. 3, numeral 60 is a control part, to which the laser source 9, the footswitch 7, the control box 3, each sensor, each driving device, and others are connected. In the control box 3, there are provided a rotary knob 3a for setting laser output power of the treatment beam, a switch 3b for setting a light quantity of the aiming beam, a color switch 3c for selecting (setting) a wavelength (color) of the treatment beam and the aiming beam, and a switch 3d for switching an operating mode of the apparatus between a laser irradiation enabled state (a READY mode) and a laser irradiation disabled state (a STANDBY mode). In addition, the control box 3 is provided with switches for setting photocoagulation conditions for example a duration of laser irradiation and a time interval of laser irradiation, and a display part, which are not shown in Fig. 3. The shutter 17 for the treatment beam is opened for the set irradiation duration when the footswitch 7 is depressed. The shutter 14 for the aiming beam is opened when the switch 3b is turned on (where the aiming beam is not zero).

The operation of the apparatus having the above structure is explained below.

For laser irradiation, the operator operates each switch on the control box 3 to set in advance photocoagulation conditions for example selection of a wavelength of the treatment beam and the aiming beam, laser output power, an irradiation duration. The selection of a wavelength of the treatment beam and the aiming beam is made by use of the color switch 3c to select a wavelength (red, yellow, green) adequate for a treatment purpose. In the present embodiment, the explanation is made assuming that the yellow laser beam is selected. After the selection of the wavelength, the laser beam of a selected wavelength is emitted from the laser source 9. The operator presses the switch 3d to change the operating mode of the apparatus from the STANDBY mode to the READY mode, thereby opening the safety shutter 10. When the switch 3b is turned on, furthermore, the shutter 14 is opened by the driving device 64, which allows only the S-polarized beam utilized as the aiming beam split by the polarizer 11 to travel through the optical fiber 2 and be delivered to the laser irradiating part 5 of the slit lamp delivery 4. Thus, the aiming beam is irradiated to the eye fundus.

The operator observes the fundus of the patient's eye and the aiming beam through the slit lamp delivery 4 to make alignment of the aiming beam with respect to the affected part. Succeedingly, when the operator depresses the footswitch 7, the shutter 17 is opened. This allows the P-polarized beam utilized as the treatment beam to pass through the polarizer 16 and be combined with the S-polarized beam utilized as the aiming beam, and then the combined laser beam is delivered to the laser irradiating part 5 through the optical fiber 2. Thus, the treatment beam and the aiming beam are irradiated to the eye fundus. The control part 60 controls the output power of the laser source 9 so that the laser output power of the treatment beam and the quantity of the aiming beam are adjusted to the settings determined by the use of the rotary knob 3a and the switch 3b on the control box 3, respectively.

In the above apparatus, the aiming beam of the same color (wavelength) as that of the treatment beam is used for alignment, which enables observation of the transmittance property of the actual treatment beam. The yellow laser beam is selected in the above explanation; however, if the transmittance property become largely different depending on laser wavelengths, the operator selects an appropriate laser beam from among green, yellow, and red laser beams by observing each transmittance property.

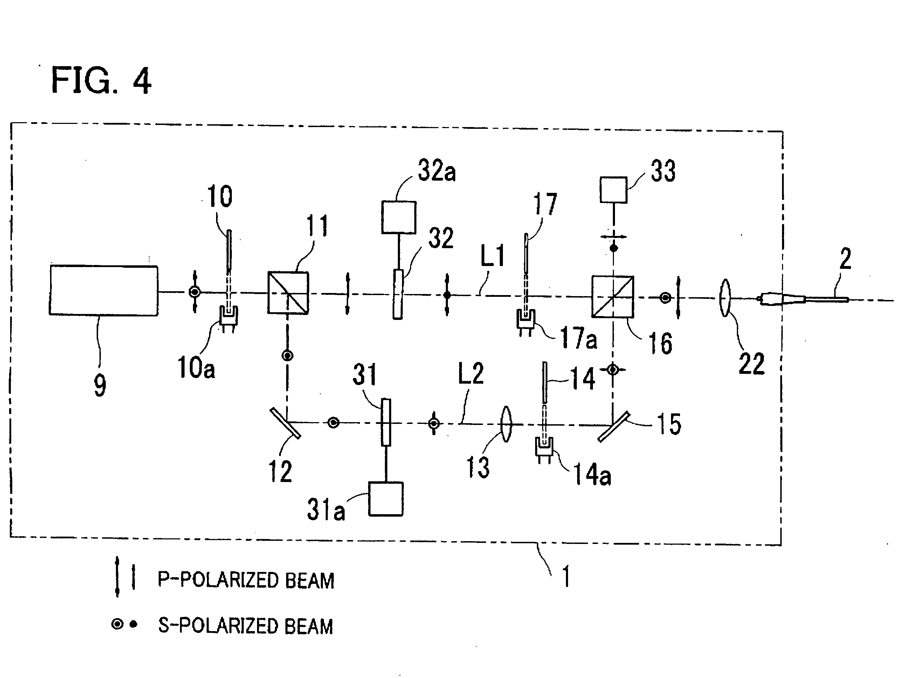

Fig. 4 is a schematic view showing a modification example of the optical system of the apparatus. In some cases, the solid laser such as an Nd:YAG laser may provide more satisfactory stability when the laser is operated at a fixed output power. In this case, as the modification example shown in Fig. 4, a 1/2 wave plate 32 is disposed on the optical axis L1 and another 1/2 wave plate 31 is disposed on the optical axis L2, so that respective light quantities (powers) of the treatment beam and the aiming beam can be controlled. In Fig. 4, like elements corresponding to those of the optical system shown in Fig. 2 are indicated by like numerals.

When the 1/2 wave plates 31 and 32 are rotated, a polarization plane of each beam which passes through each plate is rotated. Accordingly, a polarization ratio (P/S) in each beam can be freely changed. The polarizer 16 combines only the P-polarized beam passed through the 1/2 wave plate 32 and the S-polarized beam passed through the 1/2 wave plate 31 and directs the combined laser beam into the optical fiber 2. More specifically, the polarizer 16 not only serves to combine the P-polarized beam for treatment and the S-polarized beam for aiming but also serves as an attenuator in combination with the 1/2 wave plates 31 and 32 to control each light quantity. It is to be noted that the S-polarized beam passed through the 1/2 wave plate 32 is reflected by the polarizer 16 and the P-polarized beam passed through the 1/2 wave plate 31 is allowed to pass through the polarizer 16, and both the polarized beams come into a diffuser 33. That is, the diffuser 33 serves to absorb the laser beam no longer required in order to reduce outputs of the treatment beam and the aiming beam.

The laser output power of the treatment beam is set with the rotary knob 3a and the light quantity of the aiming beam is set with the switch 3b. The 1/2 wave plate 32 is rotated by a driving part 32a and the 1/2 wave plate 31 is rotated by a driving part 31a.

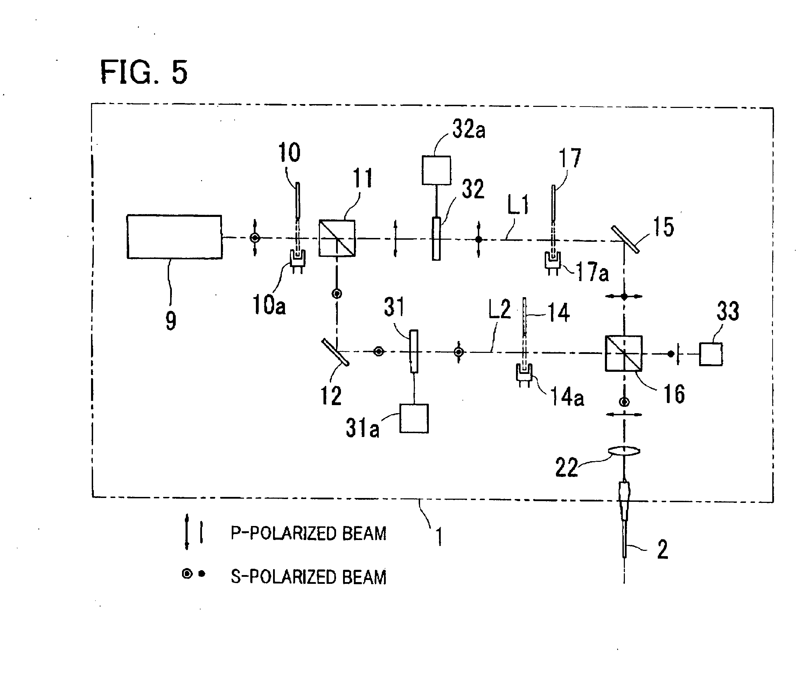

Fig. 5 is a schematic view showing another modification example of the optical system shown in Fig. 4. This optical system is arranged such that the mirror 15 and the polarizer 16 in the optical system of Fig. 4 are interchanged to provide the optical paths of equal length between the polarizers 11 and 16. The optical paths of equal length can eliminate the need of the compensating lens 13 disposed on the optical axis L2.

In the above embodiment, a polarizing filter may be provided instead of the 1/2 wave plate 31 disposed on the optical axis L2. In this case, the polarizing filter is driven to rotate, thereby attenuating the light quantity of the aiming beam to control the light quantity. Instead of the 1/2 wave plate 31, alternatively, a variable density filter which continuously varies optical density clockwise may be provided. In this case, the variable density filter is driven to rotate, thereby attenuating the light quantity of the aiming beam to control the light quantity.

Furthermore, a brewster plate may be used as the polarizer. This brewster plate has an advantage of causing little loss with respect to linearly polarized light.

The polarization ratio of the S-polarized beam for aiming to the P-polarized beam for treatment which are split by the polarizer 11 has only to be in just about the same range as an attenuation ratio of an attenuation filter conventionally used, so that the above structure can be used effectively.

Although the S-polarized beam is utilized as the aiming beam in the above embodiment, instead thereof, the P-polarized beam may be utilized as the aiming beam if the P-polarized beam becomes lower in value of the polarization ratio than the S-polarized beam in association with the linear polarization of the laser beam from the laser source and the placement of the polarizer 11.

As described above, according to the present invention, an aiming beam of the same wavelength (color) as the treatment laser beam can be obtained with a low loss by a simple manner. In addition, the mechanism for controlling output power of the treatment laser beam and the aiming beam can economically be structured.

While the presently preferred embodiment of the present invention has been shown and described, it is to be understood that this disclosure is for the purpose of illustration and that various changes and modifications may be made without departing from the scope of the invention as set forth in the appended claims.

## Claims

1. A laser treatment apparatus for performing treatment by irradiating an affected part with a laser beam including
a laser source (9) which emits a laser beam having a wavelength in a visible wavelength region, **characterized by**
a polarization splitter member (11) which splits the laser beam emitted from the laser source into a P-polarized component and an S-polarized component; and
a polarization combining member (16) which combines optical axes of the split components in a predetermined positional relation.

2. The laser treatment apparatus according to claim 1, wherein the polarization combining member (16) combines the optical axes of the split components in a coaxial relation.

3. The laser treatment apparatus according to claim 1 or 2, further including a shutter (17) which is removably disposed on one of the optical axes which is higher in a polarization ratio between the split components.

4. The laser treatment apparatus according to one of claims 1 to 3, further including a half-wavelength plate (31, 32) rotatably disposed on at least one of the optical axes of the split components.

5. The laser treatment apparatus according to one of claims 1 to 4, wherein the laser source (9) emits a plurality of laser beams having different wavelengths in the visible wavelength region.

6. The laser treatment apparatus according to one of claims 1 to 5, wherein the polarization splitter member (11) includes a brewster plate.

## Patentansprüche

1. Laserbehandlungsgerät zum Ausführen einer Behandlung durch Bestrahlen eines befallenen Teiles mit einem Laserstrahl, mit
einer Laserquelle (9), die einen Laserstrahl mit einer Wellenlänge in einem sichtbaren Wellenlängenbereich emittiert,
**gekennzeichnet durch**
ein Polarisationsteilerteil (11), das den von der Laserquelle emittierten Laserstrahl in eine P-polarisierte Komponente und eine S-polarisierte Komponente teilt; und
ein Polarisationskombinierteil (16), das optische Achsen der geteilten Komponente in einer vorbestimmten Positionsbeziehung kombiniert.

2. Laserbehandlungsgerät nach Anspruch 1, bei dem das Polarisationskombinierteil (16) die optischen Achsen der geteilten Komponente in einer koaxialen Beziehung kombiniert.

3. Laserbehandlungsgerät nach Anspruch 1 oder 2, weiter mit einem Verschluß (17), der entfernbar in einer der optischen Achsen vorgesehen ist, die höher in dem Polarisationsverhältnis zwischen den geteilten Komponenten ist.

4. Laserbehandlungsgerät nach einem der Ansprüche 1 bis 3, weiter mit einer Halbwellenlängenplatte (31, 32), die drehbar auf mindestens einer der optischen Achsen der geteilten Komponenten vorgesehen ist.

5. Laserbehandlungsgerät nach einem der Ansprüche 1 bis 4, bei dem die Laserquelle (9) eine Mehrzahl von Laserstrahlen mit verschiedenen Wellenlängen in dem sichtbaren Wellenlängenbereich emittiert.

6. Laserbehandlungsgerät nach einem der Ansprüche 1 bis 5, bei dem das Polarisationsteilerteil (11) eine Brewster-Platte enthält.

## Revendications

1. Dispositif de traitement par laser destiné à exécuter un traitement en irradiant une partie affectée avec un faisceau laser, comprenant
une source laser (9) qui émet un faisceau laser ayant une longueur d'onde dans un domaine de longueur d'onde du visible, **caractérisé par**
un élément de séparateur de polarisation (11) qui sépare le faisceau laser émis à partir de la source laser en une composante à polarisation P et une composante à polarisation S, et
un élément de combinaison de polarisation (16) qui combine les axes optiques des composantes séparées dans une relation de position prédéterminée.

2. Dispositif de traitement par laser selon la revendication 1, dans lequel l'élément de combinaison de polarisation (16) combine les axes optiques des composantes séparées en une relation coaxiale.

3. Dispositif de traitement par laser selon la revendication 1 ou 2, comprenant en outre un obturateur (17) qui est disposé de façon amovible sur l'un des axes optiques qui présente un rapport de polarisation supérieur entre les composantes séparées.

4. Dispositif de traitement par laser selon l'une des revendications 1 à 3, comprenant en outre une lame demi-onde (31, 32) disposée avec possibilité de rotation sur au moins l'un des axes optiques des composantes séparées.

5. Dispositif de traitement par laser selon l'une des revendications 1 à 4, dans lequel la source laser (9) émet une pluralité de faisceaux laser ayant des longueurs d'onde différentes dans le domaine de longueur d'onde du visible.

6. Dispositif de traitement par laser selon l'une des revendications 1 à 5, dans lequel l'élément de séparateur de polarisation (11) comprend une lame de Brewster.
